Europäisches Patentamt

European Patent Office

Office européen des brevets

(11) Veröffentlichungsnummer: **0 303 810**
**A2**

(12) **EUROPÄISCHE PATENTANMELDUNG**

(21) Anmeldenummer: **88110641.3**

(22) Anmeldetag: **04.07.88**

(51) Int. Cl.⁴: **A61B 17/56**

(30) Priorität: **15.08.87 DE 8711113 U**

(43) Veröffentlichungstag der Anmeldung:
**22.02.89 Patentblatt 89/08**

(84) Benannte Vertragsstaaten:
**AT BE CH DE ES FR GB GR IT LI LU NL SE**

(71) Anmelder: **orthoplant Endoprothetik GmbH**
**Leerkämpe 12**
**D-2800 Bremen 66(DE)**

(72) Erfinder: **Schelhas, Klaus-Dieter**
**Leerkämpe 12**
**D-2800 Bremen 66(DE)**

(74) Vertreter: **Eisenführ & Speiser**
**Martinistrasse 24**
**D-2800 Bremen 1(DE)**

(54) **Vorrichtung zum Schneiden eines Gewindes in einem pfannenförmigen Knochen.**

(57) Es wird eine Vorrichtung zum Schneiden eines Gewindes in einem pfannenförmigen Knochen, insbesondere im Acetabulum eines menschlichen Beckenknochens angegeben, die einen Schneidenhalter an einer Antriebswelle enthält, wobei an dem Schneidenhalter ein radial nach aussen gerichtetes Schneidmesser angeordnet ist. Um in einfacher Weise ein maßhaltiges Gewinde in das Knochengewebe mit gewünschter Schnittiefe einzuschneiden, enthält die Vorrichtung eine im Pfannenraum fixierbare Schneidlehre mit einer zentralen Bohrung und einem um die Längsachse der Bohrung schraubenförmig verlaufenden, offenen Schneidkanal. Der Schneidenhalter ist zentral in die Bohrung der Schneidlehre einführbar und mit dem Einführen des Messers in den Schneidkanal in eine zwangsgeführte schraubenförmige Schneidbewegung versetzbar, im Verlauf derer das Schneidmesser das Gewinde präzise in das Knochengewebe schneidet.

FIG. 3

## Vorrichtung zum Schneiden eines Gewindes in einem pfannenförmigen Knochen.

Die Erfindung betrifft eine Vorrichtung zum Schneiden eines Gewindes in einem pfannenförmigen Knochen, insbesondere im Acetabulum eines menschlichen Beckenknochens, mit einem Schneidenhalter an einer Antriebswelle und mit einem radial nach außen gerichteten Schneidmesser an dem Schneidenhalter.

Aus der DE-OS 28 34 296 ist eine derartige Vorrichtung bekannt, bei welcher der Schneidenhalter eine den Pfannenhohlraum ausfüllende halbkugelförmige Gestalt und ein radial ausfahrbares Schneidmesser aufweist, welches zum Schneiden von hinterdrehten Nuten im Acetabulumgewebe des menschlichen Beckenknochens dient. Da jedoch das Knochengewebe des menschlichen Acetabulum in verschiedenen Teilen der Oberfläche unterschiedlich hart ist und insbesondere in der Stützzone des Oberschenkelkopfes besonders sklerodiert ist, wird das Schneidmesser bevorzugt in den weichen Oberflächenbereichen in das Knochengewebe eindringen; dagegen wird das besonders harte Knochengewebe das Schneidmesser aus dem Knochen herausdrängen und damit auch den Schneidenhalter im Acetabulum in eine Fehlposition bringen, so daß der erzeugte Schnitt im Knochengewebe verläuft, in verschiedenen Oberflächenbereichen ungleich tief und insgesamt wenig maßhaltig ist.

Darüberhinaus sind Gewindeschneider zum Schneiden von Gewinden im Acetabulum des menschlichen Beckenknochens bekannt, die ebenfalls einen das Acetabulum näherungsweise ausfüllenden Rundkörper mit einem außen umlaufenden Schneidgewinde besitzen. Aufgrund der unterschiedlichen Härte der Oberfläche des Acetabulum verrutschen auch diese Gewindeschneider aus ihrer gewünschten zentralen Arbeitsposition, derart daß das Schneidgewinde im wesentlichen nur in den weichen Bereichen des Knochengewebes eindringt, in die harten Oberflächenbereichen dagegen nicht eindringen kann. Als Resultat ergibt sich daher ein Gewinde, das in den weichen Oberflächenzonen zu tief, in den harten Oberflächenzonen mit einer zu geringen Tiefe im Knochengewebe verläuft. Wird anschließend in ein derartiges Gewinde eine künstliche Hüftpfanne mit ihrem Außengewinde eingeschraubt, so findet ein ausreichender Verbund zwischen Hüftpfanne und Knochen nur im weichen Knochengewebe statt; der gewünschte feste Verankerungsverbund zwischen künstlicher Hüftpfanne und dem Knochengewebe wird insbesondere im harten Knochengewebe nicht erzielt.

Aufgabe der Erfindung ist es demgegenüber, eine Vorrichtung der eingangs genannten Art derart weiterzubilden, daß in einfacher Weise ein maßhaltiges Gewinde in das Knochengewebe einschneidbar ist, welches durchgängig die gewünschte Schnittiefe besitzt.

Diese Aufgabe wird bei der Vorrichtung der eingangs genannten Art erfindungsgemäß dadurch gelöst, daß eine im Pfannenraum fixierbare Schneidlehre mit einer zentralen Bohrung und einem um die Längsachse der Bohrung schraubenförmig verlaufenden, offenen Schneidkanal vorgesehen ist, und daß der Schneidenhalter zentral in die Bohrung der Schneidlehre einführbar ist und mit dem Einführen des Schneidmessers in den Schneidkanal in eine zwangsgeführte schraubeförmige Schneidbewegung versetzbar ist.

Die Vorteile der Erfindung liegen insbesondere darin, daß eine den Pfannenhohlraum füllende Schneidlehre im Acetabulum fixiert wird, und daß anschließend mit dem aus Schneidenhalter und Schneidlehre bestehenden Schneidwerkzeug ein Gewinde geschnitten wird, wobei das Schneidmesser im radial offenen Schneidkanal der Schneidlehre zwangsgeführt ist und beim Antreiben des Schneidwerkzeugs die zwangsgeführte schraubenförmige Schnittbewegung durchführt. Da die Schneidlehre im Acetabulum mechanisch oder durch zusätzliche Halteeinrichtungen von Hand unverrückbar festgehalten wird, ist ein Verlaufen oder Verdrängen des Schneidmessers in den unterschiedlich harten Knochenzonen nicht möglich. Das Schneidmesser wird vielmehr die vom Schneidkanal exakt vorgegebene Zwangsbahn entlanglaufen und dabei ein maßhaltiges Gewinde mit der jeweils eingestellten Schnittiefe erzeugen, zumal während eines Schneidvorganges der Schneidenhalter in der zentralen Bohrung der Schneidlehre mit geringem Spiel so geführt ist, daß er die vom Schneidmesser erzwungene Schraubbewegung zentrisch exakt durchführen kann.

Besonders bevorzugt ist das Schneidmesser im Schneidenhalter radial verschiebbar gelagert, und die Radialposition des Schneidmessers läßt sich mit Einstellelementen verstellen und fixieren. Mit einer derartigen Vorrichtung ist es dann möglich, auch Gewinde mit besonders großer Schnittiefe in mehreren aufeinanderfolgenden Schneidvorgängen genau zu verwirklichen, wobei das Schneidmesser für jeden nachfolgenden Schneidvorgang weiter radial auswärts eingestellt und fixiert wird, so daß die endgültige Schnittiefe in Schritten ohne Auswechslung des Schneidwerkzeuges erreicht wird. Diese Ausführungsform der Erfindung ist besonders vorteilhaft beim Schneiden des zum Einsetzen von künstlichen Hüftpfannen benötigten Gewindes im Acetabulum, weil insbesondere die Zonen mit hartem Knochengewebe das Schneiden von Gewindegängen mit der gewünschten endgültigen Schnittiefe in einem Schritt nicht zulassen.

Besonders bevorzugt besitzt die Schneidlehre eine dem Pfannenhohlraum des Acetabulum angepasste kalottenförmige Außenform, die auf der Außenfläche nach außen gegen den Knochen gerichtete Fixierstifte besitzt. Zum Fixieren der Schneidlehre wird diese in das Acetabulum eingesetzt und anschließend - z.B. unter dem leichten Schlag eines Hammers - mit den Fixierstiften im Knochen verankert. Zusätzlich können an der Schneidlehre noch Befestigungsmittel, z.B. Gewindebuchsen etc. angebracht sein, an denen sich Haltegriffe ansetzen lassen, so daß die Schneidlehre noch zusätzlich von Hand gehalten werden kann.

Besonders bevorzugt besitzt der Schneidenhalter eine in die Bohrung der Schneidlehre passende zylindrische Form in koaxialer Verlängerung der Antriebswelle. Außen auf dem Schneidenhalter ist ein um dessen zentrale Achse verlaufendes Außengewinde vorgesehen, dessen Steigung der Steigung des Schneidkanals der Schneidlehre ist, so daß das Außengewinde des Schneidenhalters bei oder nach dem Einführen in die Schneidlehre im Schneidkanal geführt ist und dadurch eine genaue Führung des Schneidenhalters während der gesamten Schneidbewegung bewirkt.

Bevorzugt ist das freie Ende des Schneidenhalters kalottenförmig ausgebildet und dient beim Anlaufen gegen den Boden des Pfannenhohlraums als Anschlag, der ein weiteres Eindrehen des Schneidenhalters verhindert und damit den Schneidvorgang begrenzt. Zusätzlich kann in der Schneidlehre auch noch mindestens ein mechanischer Anschlag vorgesehen werden, gegen welchen der Schneidenhalter oder das Schneidmesser am Ende einer Schneidbewegung anlaufen und dadurch den Schneidvorgang beenden kann ohne den Beckenknochen zu beschädigen.

Das Schneidmesser ist bevorzugt am kalottenförmigen Ende des Schneidenhalters in einer radialen Nut radial verschiebbar gelagert und besitzt in radialer Richtung eine Zahnung, die mit einem im Schneidenhalter gelagerten Stellritzel zusammenwirkt. Das Stellritzel ist bevorzugt mit einem Einstellelement gekoppelt, an welchem der Operateur die Position, und damit die Schnittiefe des Schneidmessers einstellen kann. Als Einstellelement ist bevorzugt eine Hohlwelle um die Antriebswelle vorgesehen, die mit einer Außenzahnung mit dem Stellritzel im Schneidenhalter zusammenwirkt. Die Hohlwelle läßt sich mit einem einförmigen Eingriff verwehen, um eine einfache Herstel-lung des Schneidmessers zu ermöglichen. Zwischen der Hohlwelle und der Antriebswelle sind Rastmittel zu lösbaren Fixieren der Radialstellung vorhanden, die bevorzugt eine Umfangszahnung an der Antriebswelle oder an der Hohlwelle und ein mit der Umfangszahnung zusammenwirkendes, federbelastetes Rastelement enthalten.

Besonders bevorzugt besitzt die erfindungsgemäße Vorrichtung Mittel zum Zu- und Abführen einer Spüllösung an die Schneidstelle. Zu diesem Zweck ist die Antriebswelle bevorzugt als eine doppelwandige Koaxial-Hohlwelle ausgebildet, die zwischen der äußeren und inneren Hohlwelle eine Ringkammer zum Einströmen der Spüllösung, z.B. einer Kochsalzlösung, und in der inneren Hohlwelle einen Kanal zum Ableiten der Spüllösung enthält, wobei in vorgegebenen Abstand von dem Schneidenhalter eine Ein- und Ausspeisekupplung vorgesehen ist, die mit einem Ringkanal mit dem ringförmigem Einströmkanal, und mit einem weiteren Ringkanal mit dem Abströmkanal verbunden ist.

Ganz besonders bevorzugt ist an dem dem Schneidenhalter gegenüberliegenden Ende der Antriebswelle eine Betätigungskurbel z.B. mittels eines Spannfutters an der Antriebswelle befestigbar. Die Antriebswelle bzw. die innere Hohlwelle der Antriebswelle ist mit dem Schneidenhalter fest oder auch lösbar verbindbar. Die äußere Hohlwelle der Antriebswelle sowie die als Einstellmittel für das Schneidmessers dienende äußere Hohlwelle lassen sich - wie das Stellritzel - und wie die Kupplung zum Ein- und Ausspeisen der Spüllösung auf die Antriebswelle bzw. die innere Hohlwelle der Antriebswelle aufstecken. Bevorzugt dient dann das Spannfutter dazu, die axiale Position der steckbaren Teile der Vorrichtung festzulegen.

Vorteilhafte Weiterbildungen der Erfindung sind durch die Merkmale der Unteransprüche gekennzeichnet.

Im folgenden wird ein Ausführungsbeispiel der Erfindung anhand der Zeichnung näher erläutert. Es zeigen.

Fig. 1 eine Seitenansicht der Schneidlehre;

Fig. 2 einen Schnitt durch die Schneidlehre der 1 bei eingeführtem Schneidenhalter;

Fig. 3 eine Seitenansicht des Schneidwerkzeugs, teilweise im Schnitt; und

Fig. 4 eine Aufsicht auf den Schneidenhalter gemäß Fig. 3.

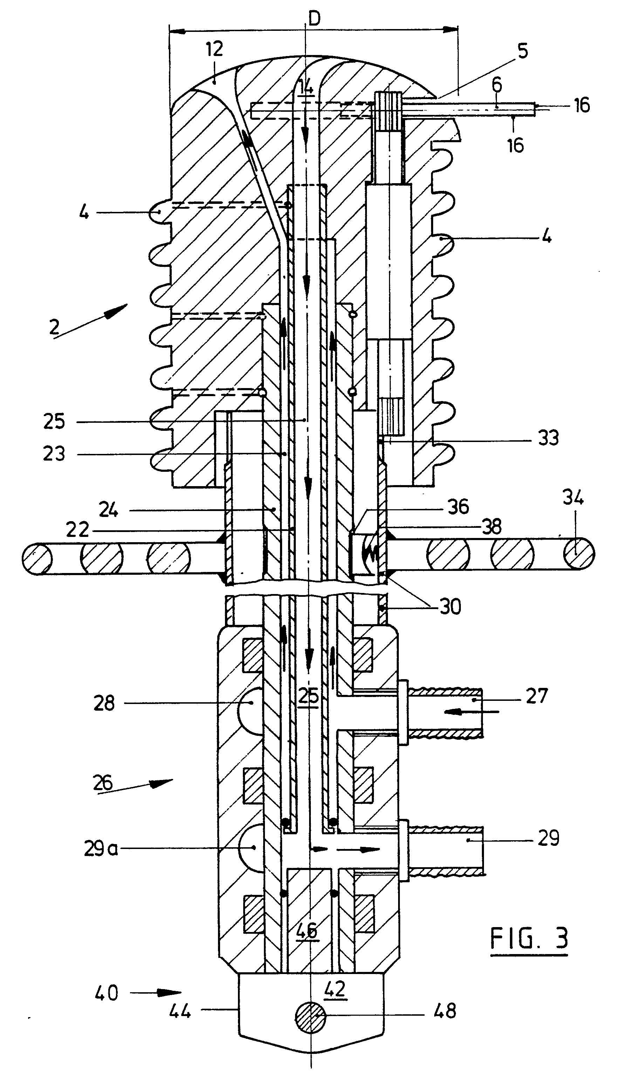

Die Fig. 1 zeigt eine Seitenansicht einer in das Acetabulum des menschlichen Beckenknochens eingesetzten Schneidlehre 50, die Bestandteil der erfindungsgemäßen Schneidvorrichtung ist.

Fig. 2 zeigt einen Schnitt durch die in Fig. 1 dargestellte Schneidlehre 50 zusammen mit dem in die Schneidlehre 50 eingesetzten Schneidwerkzeug 3. Die Schneidlehre 50 besitzt eine dem Pfannenhohlraum des Acetabulum angepasste kalottenförmige Außenform und eine zentrale Bohrung 52 mit vergleichweise großem Durchmesser. Um die Längsachse 53 der Bohrung 52 verläuft schraubenförmig ein offener Schneidkanal 54, der eine von der Bohrung bis zum Knochen reichende schraubenförmige Spaltöffnung darstellt. Die Schneidlehre besitzt auswärts gerichtete, spitze Fixierstifte 56 auf ihrer Außenfläche und einen Anschlag 58 am oberen Ende des Schneidkanals 54. An der in Pfannenöffnung liegende untere Grundfläche 59 der Schneidlehre sind Befestigungsmittel 60 angeschweißt, die z.B. ein Innengewinde zum Anschrauben von Haltengriffen besitzt.

Fig. 2 zeigt einen Querschnitt durch die in das Acetabulum eingesetzte Schneidlehre 50. Dargestellt ist außerdem das Schneidwerkzeug 3, welches einen Schneidenhalter 2 enthält, an dem zentral eine Antriebswelle 4 befestigt ist. Der Schneidenhalter besitzt eine in die Bohrung 52 der Schneidlehre 50 passende zylindrische Form sowie ein um seine Symmetrieachse verlaufendes Außengewinde, desssen Steigung gleich der Steigung des Schneidkanals 54 ist. Das freie Ende des Schneidenhalters ist konvex abgerundet und besitzt eine radial verlaufende Führungsnut 5, in der ein Schneidmesser 6 radial gerichtet und radial verschiebbar gelagert ist, wobei das Schneidmesser 6 im Außengewinde bzw. in Verlängerung der Schraubenlinie des Außengewindes 4 angeordnet ist, so daß der aus dem Schneidenhalter 2 herausragende Teil des Schneidmessers 6 ebenfalls in dem Schneidkanal 54 geführt ist, wenn das Außengewinde 4 in den Schneidkanal 54 eingeschraubt wird. Alternativ ist es auch möglich auf das Außengewinde 4 zu verzichten. Der Schneidenhalter 2 wird dann in der Bohrung 52 geführt während das Schneidmesser in dem Schneidkanal seine schraubenförmige, zwangsgeführte Schneidbewegung durchführen kann, wenn die Antriebswelle in eine Drehbewegung versetzt wird.

Der Schneidvorgang wird wegen der besonderen Härte des den Pfannenhohlraum umgebenden Knochengewebes in mehreren Schritten bis zur endgültigen Schnittiefe durchgeführt.In Fig. 2 ist daher das beschnittene Gewinde während eines zweiten Schneidvorganges dargestellt, der mit dem Durchmesser $D_2$ erfolgt. Der obere Teil des beschnittenen Gewindes wurde in dem vorausgegangenen ersten Schneidvorgang mit einem Durchmesser $D_1$ geschnitten, weitere Schneidvorgänge mit größeren Durchmessern und entsprechend weiter ausgefahrenem Schneidmesser 6 können sich anschließen.

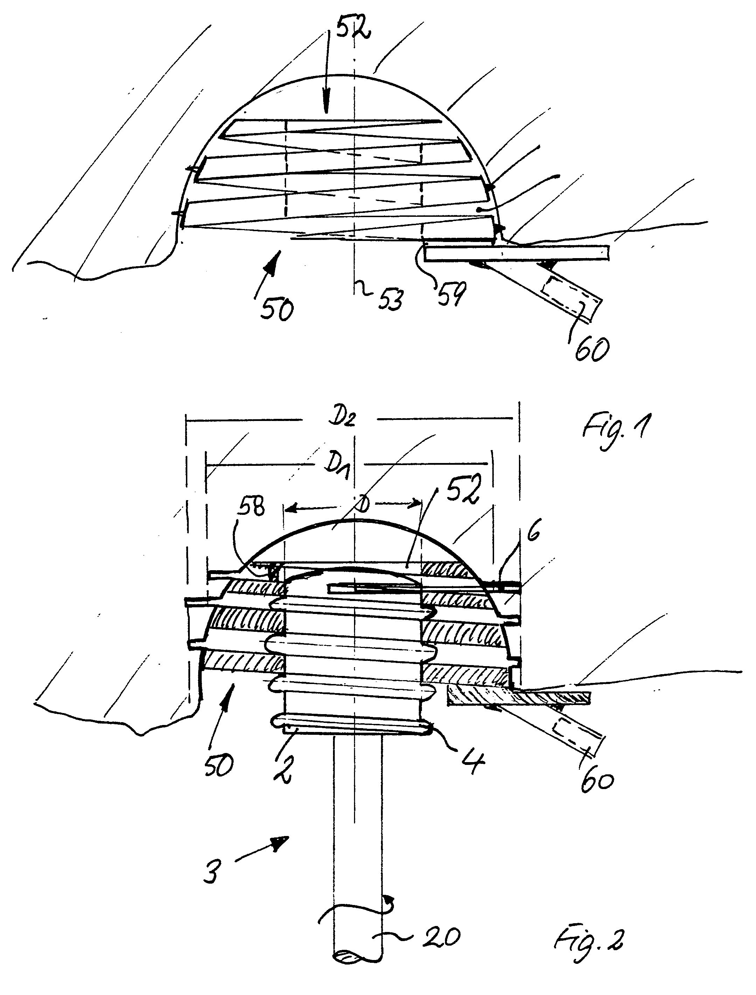

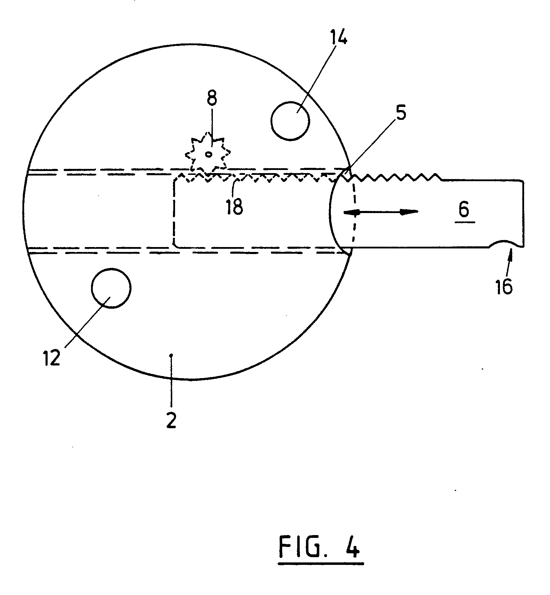

Die Figs. 3 und 4 zeigen einen Schnitt bzw. eine Aufsicht auf das Schneidwerkzeug 3. Der Schneidenhalter 2 besitzt die Form eines in die Bohrung 52 der Schneidlehre 50 passenden Zylinderkörpers, mit Außengewinde 4 um die Symmetrieachse des Schneidenhalters, in deren Verlängerung die Antriebswelle 20 verläuft und am Schneidenhalter befestigt ist. Der Schneidenhalter weist an seinem freien Ende eine radiale Führungsnut 5 auf, in welcher das Schneidmesser 6 radial verschiebbar gelagert ist, vgl. Fig. 4. Das Schneidmesser 6 besitzt mindestens an seinem radial ausfahrbaren Ende eine Schneidkante 16, es besitzt außerdem in radialer Richtung eine Zahnung 18, die mit einem Stellritzel 8 zusammenwirkt, welches im Schneidenhalter 2 parallel zur Achse der Antriebswelle 20 drehbar gelagert ist.

Die Antriebswelle 20 besitzt eine innere Hohlwelle 22 und beabstandet hierzu eine äußere Hohlwelle 24, die beide drehfest mit dem Schneidenhalter 2 verbunden sind. Eine weitere Hohlwelle 30 ist um die beiden Hohlwellen 22, 24 der Antriebswelle angeordnet und dient als Einstellelement, welches mittels einer Außenzahnung 33 mit dem Stellritzel 8 zusammmenwirkt, das in die Zahnung 18 des Schneidmessers 6 greift und das Schneidmesser radial verstellt. An der Hohlwelle 30 ist ein ringförmiger Betätigungsgriff 34 befestigt. Die Hohlwelle 30 oder die Antriebswelle 20 besitzt eine Umfangzahnung 36, in die ein federbelastetes Rastmittel 38 greift, um die eingestellte Radialposition des Schneidmessers 6 lösbar zu sichern.

An die Hohlwelle 30 schließt axial eine auf der Antriebswelle 20 sitzende Rotationskupplung 26 an, die an einem ersten Stutzen 27 in einen ersten Ring kanal 28 übergeht, der in dem Ringkanal 23 zwischen den Hohlwelle mündet. Der Kanal 23 geht im Schneidenhalter 2 in einen Einströmkanal 12 über, der am freien Ende des Schneidenhalters mündet. Die Rotationskupplung 26 geht von einem zweiten Stutzen 29 in einen zweiten Ringkanal 29 über, der in dem zentralen Kanal 25 der inneren Hohlwelle 22 mündet. Der Kanal 25 geht im Schneidenhalter 2 in einen zentralen Abströmkanal 14 über, der ebenfalls am freien Ende des Schneidenhalters 2 austritt. Von einem Reservoir läßt sich über den Kanal 27, 28, 23,12 Spüllösung an die Schneidstelle führen und über den Kanal 14, 25, 29a, zusammen mit den Schneidspänen wieder ableiten.

An dem dem Schneidenhalter 2 gegenüberliegenden Ende der Antriebswelle 20 läßt sich über ein Spannfutter 40 eine Betätigungskurbel 48 (nur teilweise dargestellt) befestigen, wobei ein geschlitztes Innenfutter 42 mittels einer Überwurfmutter 44 auf die Antriebswelle 20 aufgezogen wird. Das Ritzel 8, die Hohlwelle 30, ggf. die Hohlwellen 22 und 24 der Antriebswelle sowie die Rotationskupplung 26 lassen sich deckbar und lösbar miteinander verbinden; in diesem Fall dient das Spannfutter 40 dann zur Festlegung der Axialpositionen der einzelnen Teile. Die steckbare Verbindung der Einzelteile bewirkt, daß das Schneidwerkzeug leicht auseinander genommen, gereinigt und wieder zusammen gebaut werden kann.

## Ansprüche

1. Vorrichtung zum Schneiden eines Gewindes in einem pfannenförmigen Knochen, insbesondere im Acetabulum eines menschlichen Beckenknochens, mit einem Schneidenhalter an einer Antriebswelle und mit einem radial nach außen gerichteten Schneidmesser an dem Schneidenhalter, dadurch gekennzeichnet, daß eine im Pfannenraum fixierbare Schneidlehre (50) mit einer zentralen Bohrung (52) und einem um die Längsachse der Bohrung (52) schraubenförmig verlaufenden, offenen Schneidkanal (54) vorgesehen ist, und daß der Schneidenhalter (2) zentral in die Bohrung (52) der Schneidlehre (50) einführbar ist und mit dem Einführen des Schneidmessers (6) in den Schneidkanal (54) in eine zwangsgeführte schraubenförmige Schneidbewegung versetzbar ist.

2. Vorrichtung nach Anspruch 1, dadurch gekennzeichnet, daß das Schneidmesser (6) radial verschiebbar in den Schneidenhalter (2) gelagert ist.

3. Vorrichtung nach Anspruch 1 oder 2, dadurch gekennzeichnet, daß der Schneidenhalter (2) eine in die Bohrung (52) der Schneidlehre (50) passende zylindrische Form, und ein um die Achse der Antriebswelle verlaufendes Außengewinde (4) besitzt, welches bei oder nach dem Einführen des Schneidmessers (6) in den Schneidkanal (54) im Schneidkanal (54) verschraubbar ist.

4. Vorrichtung nach einem der vorsteheden Ansprüche, dadurch gekennzeichnet, daß die Schneidlehre (50) eine dem Pfannenhohlraum angepasste kalottenförmige Außenform besitzt.

5. Vorrichtung nach einem der vorstehenden Ansprüche, dadurch gekennzeichnet, daß der Schneidenhalter (2) an seinem freien Ende kalottenförmig ausgebildet ist.

6. Vorrichtung nach einem der vorstehenden Ansprüche, dadruch gekennzeichnet, daß das Schneidmesser (6) am kalottenförmigen Ende des Schneidenhalters (2) angeordnet ist.

7. Vorrichtung nach einem der vorstehenden Ansprüche, dadurch gekennzeichnet, daß das Schneidmesser (6) in einer radialen Nut (5) im Schneidenhalter (2) radial verschiebbar gelagert ist.

8. Vorrichtung nach einem der vorstehenden Ansprüche, dadurch gekennzeichnet, daß das Schneidmesser (6) in Radialrichtung eine Zahnung (18) besitzt, die mit einem im Schneidenhalter (2) gelagerten Stellritzel (8) zusammen wirkt und mittels eines Einstellelementes (30) verstellbar ist.

9. Vorrichtung nach Anspruch 8, dadurch gekennzeichnet, daß das Stellritzel (8) parallel zur Achse der Antriebswelle (20) gelagert ist, und daß das Einstellelement (30) eine um die Antriebswelle (20) angeordnete Hohlwelle (32) mit einem Betätigungsgriff (34) und mit einer mit dem Stellritzel (8) zusammenwirkenden Außenzahnung (33) ist.

10. Vorrichtung nach Anspruch 9, dadurch gekennzeichnet, daß zwischen dem Einstellelement (30) und der Antriebswelle (20) Rastmittel zum lösbaren Fixieren der Radialstellung des Schneidmessers (6) vorgesehen sind.

11. Vorrichtung nach einem der vorstehenden Ansprüche, dadurch gekennzeichnet, daß die Schneidlehre (50) auswärts gegen den Knochen gerichtete Fixierstifte (56) besitzt.

12. Vorrichtung nach einem der vorstehenden Ansprüche, dadurch gekennzeichnet, daß die Schneidlehre (50) zur Begrenzung der Schneidbewegung des Schneidmessers (6) im Schneidkanal (54) mindestens einen Anschlag (58) enthält.

13. Vorrichtung nach einem der vorstehenden Ansprüche, gekennzeichnet durch Mittel (10, 12, 22, 24, 26) zum Zu- und Abführen einer Spüllösung an die Schneidstelle.

14. Vorrichtung nach Anspruch 13, dadurch gekennzeichnet, daß die Mittel zum Zu- und Abführen der Spüllösung in der Antriebswelle (20) und dem Schneidenhalter (2) verlaufende Einströmkanäle (23, 12) und Abströmkanäle (24, 14) und eine Ein- und Ausspeisekupplung (26) an der Antriebswelle (20) enthalten.

15. Vorrichtung nach einem der vorstehenden Ansprüche, dadurch gekennzeichnet, daß an dem dem Schneidenhalter (2) gegenüberliegenden Ende der Antriebswelle (20) eine Betätigungskurbel (48) befestigbar ist.

16. Vorrichtung nach Anspruch 15, dadurch gekennzeichnet, daß die Betätigungkurbel (48) mittels eines Spannfutters (40) an der Antriebswelle (20) befestigbar ist.

17. Vorrichtung nach einem der vorstehenden Ansprüche, dadurchgekennzeichnet, daß die Hohlwelle (32) sowie die Speisekupplung (28) für die Spüllösung und ggf. das Ritzel (8) auf den Schneidenhalter (2) und/oder die Antriebswelle (2) aufsteckbar und mittels des Spannfutters (40) in ihrer axialen Position festsetzbar sind.

18. Vorrichtung nach einem der vorstehenden Ansprüche,
dadurch gekennzeichnet, daß an der Schneidlehre (50) Befestigungsmittel (60) zum Ansetzen von Haltegriffen vorgesehen sind.

FIG. 3

Fig. 1

Fig. 2

FIG. 4